# FASCICULE DE BREVET EUROPEEN

(11) **EP 0 667 142 B1**
(45) Date de publication et mention de la délivrance du brevet: **18.06.1997**
(21) Numéro de dépôt: 94402664.0
(22) Date de dépôt: 22.11.1994
(51) Int. Cl.: A61K 7/13

(54) **Composition de teinture d'oxydation des fibres kératiniques comprenant un dérivé de paraphénylènediamine et le 2-méthyl 5-aminophénol, et procédé de teinture utilisant une telle composition**
Paraphenylenediaminederivat und 2-methyl-5-aminophenol enthaltende Oxydationsfärbezusammensetzung und Färbeverfahren mit dieser Zusammensetzung
Oxidation dyeing composition for keratinic fibers comprising a paraphenylenediamine derivative and 2-methyl-5-aminophenol and method using such a composition

(30) Priorité: 24.01.1994 FR 9400703
(43) Date de publication de la demande: 16.08.1995
(73) Titulaire: L'OREAL, 75008 Paris (FR)
(72) Inventeur: Audousset, Marie-Pascale, F-92600 Asnieres (FR); Cotteret, Jean, F-78480 Verneuil s/Seine (FR)
(74) Mandataire: Andral, Christophe André Louis

(56) Documents cités:
- EP-A- 0 007 537
- EP-A- 0 400 330
- EP-A- 0 634 163
- EP-A- 0 634 164
- WO-A-91/09587
- DE-A- 3 627 398
- FR-A- 2 364 888
- PATENT ABSTRACTS OF JAPAN vol. 4 no. 174 (C-033) ,2 Décembre 1980 & JP-A-55 115814 (LION CORP.) 6 Septembre 1980,

## Description

La présente invention concerne une composition de teinture d'oxydation des fibres kératiniques et en particulier des fibres kératiniques humaines, comprenant en association, au moins la 2-(β-hydroxyéthyl) paraphénylènediamine avec le 2-méthyl 5-aminophénol. Elle concerne également l'utilisation d'une telle composition.

Il est connu de teindre les fibres kératiniques, et en particulier les cheveux humains, avec des compositions de teinture contenant des précurseurs de colorants d'oxydation, en particulier des ortho- ou paraphénylènediamines, des ortho- ou para-aminophénols, généralement appelés "bases d'oxydation", et des coupleurs encore appelés modificateurs de coloration, plus particulièrement des métaphénylènediamines, des méta-aminophénols et des métadiphénols qui permettent de modifier et d'enrichir en reflets les colorations "de fond" obtenues par les produits de condensation des bases d'oxydation.

On recherche activement dans le domaine de la teinture capillaire d'oxydation, des précurseurs de colorants d'oxydation et des coupleurs capables d'engendrer, lorsqu'ils sont associés, une coloration rouge, ayant une résistance satisfaisante à la lumière, aux lavages, aux intempéries, à la transpiration et aux différents traitements que peuvent subir les cheveux.

Les colorants doivent également être les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Jusqu'ici ces colorations ont été obtenues avec des teintures à base de paraphénylènediamine. Mais actuellement l'utilisation de la paraphénylènediamine est remise en question pour des raisons toxicologiques.

On sait, par la demande de brevet européen EP-A-0 007 537, que l'on peut obtenir des colorations allant du pourpre au violet, en associant au moins un 1-hydroxyalkyl 2,5-diaminobenzène dans lequel le radical alkyle est en C₁-C₄, tel que le 1-hydroxyméthyl 2,5-diaminobenzène avec un coupleur pouvant notamment être le 3-amino 6-méthylphénol.

On connaît également la demande de brevet européen EP-A-634 163 ayant pour objet une composition pour la teinture d'oxydation des fibres contenant au moins un précurseur de colorant d'oxydation choisi parmi le 3-méthyl para-aminophénol, le 2-méthyl para-aminophénol et le 2-hydroxyméthyl para-aminophénol, au moins un coupleur choisi parmi des 2-méthyl 5-aminophénols substitués et au moins un précurseur de colorant d'oxydation additionnel choisi parmi les paraphénylènediamines et les bases doubles.

On connaît enfin la demande de brevet européen EP-A-634 164 concernant une composition pour la teinture des fibres contenant au moins un précurseur de colorant d'oxydation choisi parmi le 3-méthyl para-aminophénol, le 2-méthyl para-aminophénol et le 2-hydroxyméthyl para-aminophénol, au moins à titre de coupleur le 2-méthyl 5-aminophénol et au moins un précurseur de colorant d'oxydation choisi parmi les paraphénylènediamines et les bases doubles.

Ces deux demandes de brevet européen EP-A-634 163 et EP-A-634 164 ne sont opposables qu'au titre de la nouveauté.

Or, après d'importantes recherches menées sur la question, la Demanderesse vient maintenant de découvrir qu'il est possible d'obtenir de nouvelles teintures non toxiques, qui engendrent des colorations allant du rouge au pourpre, à la fois intenses et résistantes, en associant la 2-(β-hydroxyéthyl) paraphénylènediamine avec le 2-méthyl 5-aminophénol.

Cette découverte est à la base de la présente invention.

La présente invention a ainsi pour objet une composition de teinture d'oxydation pour fibres kératiniques, en particulier pour fibres kératiniques humaines telles que les cheveux, du type comprenant, dans un milieu approprié pour la teinture, au moins un précurseur de colorant d'oxydation et au moins un coupleur, et qui est caractérisée par le fait qu'elle contient, à titre de précurseur de colorant d'oxydation, au moins la 2-(β-hydroxyéthyl) paraphénylènediamine et/ou au moins l'un de ses sels d'addition avec un acide, et à titre de coupleur, le 2-méthyl 5-aminophénol et/ou au moins l'un de ses sels d'addition avec un acide, ladite composition de teinture étant exempte d'un précurseur de colorant d'oxydation additionnel qui serait choisi dans le groupe constitué par le 3-méthyl para-aminophénol, le 2-méthyl para-aminophénol et le 2-hydroxyméthyl para-aminophénol.

Les nouvelles teintures ainsi obtenues permettent d'aboutir à des nuances allant du rouge au pourpre, intenses, faiblement sélectives, ces teintures étant de surcroît des teintures non toxiques et qui résistent à la fois très bien à la lumière, aux intempéries, à la transpiration et aux différents traitements que peuvent subir les cheveux. Elles sont tout particulièrement très résistantes aux shampooings.

Un autre objet de l'invention porte sur la composition prête à l'emploi, contenant les différents agents utilisés pour la teinture des fibres kératiniques définis ci-dessus et un agent oxydant.

L'invention vise également un procédé de teinture des fibres kératiniques, et en particulier des fibres kératiniques humaines telles que les cheveux, consistant à appliquer sur ces fibres au moins une composition (A) contenant, dans un milieu approprié pour la teinture, au moins un précurseur de colorant d'oxydation et un coupleur tels qu'ils ont été définis ci-avant, la couleur étant révélée à pH alcalin neutre ou acide à l'aide d'un agent oxydant qui est ajouté juste au moment de l'emploi à la composition (A) ou qui est présent dans une composition (B) appliquée simultanément ou séquentiellement de façon séparée.

L'invention a également pour objet des dispositifs de teinture ou "kits" à plusieurs compartiments, dont le premier compartiment contient, à titre de précurseur de colorant d'oxydation, au moins la 2-(β-hydroxyéthyl) paraphénylènediamine ou l'un de ses sels d'addition avec un acide, et au moins le 2-méthyl 5-aminophénol ou l'un de ses sels d'addition avec un acide à titre de coupleur, et le deuxième compartiment un agent oxydant.

D'autres caractéristiques, aspects, objets et avantages de l'invention apparaîtront encore plus clairement à la lecture de la description et des exemples qui suivent.

Les sels d'acide qui peuvent être utilisés selon l'invention sont choisis de préférence parmi les chlorhydrates, les sulfates, les bromhydrates et les tartrates.

La concentration en précurseur de colorant d'oxydation, ou en ses sels, peut varier entre 0,01 et 10 % en poids environ par rapport au poids total de la composition de teinture, et de préférence entre 0,05 et 5 % en poids environ.

La concentration en 2-méthyl 5-aminophénol ou en ses sels peut quant à elle varier entre 0,005 et 3% en poids environ par rapport au poids total de la composition de teinture, et de préférence entre 0,05 et 2 % en poids environ.

L'agent oxydant est choisi de préférence parmi le peroxyde d'hydrogène, le peroxyde durée, les bromates de métaux alcalins, les persels tels que les perborates et les persulfates. L'utilisation du peroxyde d'hydrogène est particulièrement préférée.

La composition (A), qui renferme l'association des colorants telle que décrite ci-dessus, peut avoir un pH compris entre 3 et 11, qui peut être ajusté à la valeur choisie au moyen d'agents alcalinisants habituellement utilisés en teinture des fibres kératiniques, tels que l'ammoniaque, les carbonates alcalins, les alcanolamines telles que par les mono-, di- et triéthanolamines ainsi que leurs dérivés, les hydroxydes de sodium ou de potassium, les composés de formule : 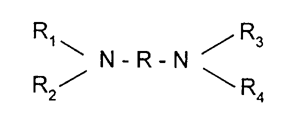 dans laquelle R est un reste propylène éventuellement substitué par un groupement hydroxyle ou un radical alkyle en C₁-C₄ ; R₁, R₂, R₃ et R₄, simultanément ou indépendamment l'un de l'autre, représentent un atome d'hydrogène, un radical alkyle en C₁-C₄ ou hydroxyalkyle en C₁-C₄,
soit au moyen d'agents acidifiants classiques, tels que les acides minéraux ou organiques comme par exemple les acides chlorhydrique, tartrique, citrique et phosphorique.

Le pH de la composition (B) renfermant l'agent oxydant tel que défini ci-dessus, est tel qu'après mélange avec la composition (A), le pH de la composition appliquée sur les fibres kératiniques humaines varie de préférence entre 3 et 11. Il est ajusté à la valeur désirée à l'aide d'agents acidifiants ou éventuellement alcalinisants bien connus de l'état de la technique, tels que ceux décrits ci-dessus.

La composition oxydante (B) est de préférence constituée par une solution d'eau oxygénée.

Selon un mode de réalisation préféré du procédé de teinture de l'invention, on mélange, au moment de l'emploi, la composition de teinture (A) décrite ci-dessus avec une solution oxydante en une quantité suffisante pour développer une coloration. Le mélange obtenu est ensuite appliqué sur les fibres kératiniques humaines et on laisse poser pendant 5 à 40 minutes, de préférence 15 à 30 minutes, après quoi on rince, on lave au shampooing, on rince à nouveau et on sèche.

Les compositions de teinture peuvent encore contenir, en plus des colorants définis ci-dessus, d'autres coupleurs et/ou des colorants directs, notamment pour modifier les nuances ou les enrichir en reflets.

Les compositions de teinture peuvent également contenir des agents antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl hydroquinone, la tertiobutyl hydroquinone et l'acide homogentisique et sont alors généralement présents dans des proportions comprises entre environ 0,05 et 1,5 % en poids par rapport au poids total de la composition.

Les compositions de teinture contiennent également, dans leur forme de réalisation préférée, des agents tensioactifs bien connus de la technique, dans des proportions comprises entre environ 0,5 et 55 % en poids, et de préférence entre 2 et 50 % en poids par rapport au poids total de la composition, des solvants organiques, dans des proportions comprises entre environ 1 et 40 % en poids, et en particulier entre 5 et 30 % en poids par rapport au poids total de la composition, ou tout autre adjuvant cosmétiquement acceptable et connu de la technique antérieure en teinture d'oxydation capillaire.

La composition appliquée sur les cheveux peut se présenter sous des formes diverses, telles que sous forme de liquide, de crème, de gel, ou sous toute autre forme appropriée pour réaliser une teinture des fibres kératiniques, et notamment des cheveux humains. En particulier, elle peut être conditionnée sous pression en flacon aérosol en présence d'un agent propulseur et former une mousse.

Des exemples concrets illustrant l'invention vont maintenant être donnés. On commencera par définir les tests utilisés pour évaluer les performances des teintures d'oxydation selon l'invention, concernant leur résistance à la transpiration, à la lumière, aux shampooings, aux intempéries ou à la permanente.

### Résistance à la transpiration :

On utilise une solution de sueur synthétique de composition suivante : 10 g de NaCl, 1 g d'hydrogénophosphate de potassium, 0,25 g d'histidine, de l'acide lactique jusqu'à pH = 3,2 et de l'eau distillée pour compléter à 100 g.

Dans un cristallisoir recouvert d'un verre de montre et renfermant cette solution de sueur, on immerge les mèches de cheveux teints qu'on laisse séjourner de 20 à 50 heures à 37° C. On rince ensuite les mèches et on les sèche.

### Résistance à la lumière (Xenotest) :

Les cheveux teints sont fixés sur un support (carton ou plastique). Ces supports sont disposés sur des porte-échantillons qui tournent autour d'une lampe Xénon pendant une durée variant de 20 à 80 heures sous un taux d'humidité variant de 25 à 75% HR (Humidité Relative) et à une température de 25° C.

### Résistance aux shampooings (machine Ahiba-Texomat) :

On place des mèches de cheveux teints dans un panier que l'on immerge dans une solution d'un shampooing standard. Le panier est soumis à un mouvement de va-et-vient vertical de fréquence variable ainsi qu'à un mouvement de rotation qui reproduisent l'action d'un frottement manuel, ce qui engendre la formation de mousse.

Après 3 minutes d'épreuve, on retire les mèches que l'on rince puis sèche. Les mèches teintes peuvent être soumises à plusieurs épreuves shampooings consécutives.

### Résistance aux intempéries (Epreuve combinée) :

Les mèches teintes sont exposées à la lumière forte ( Xenotest 40 h), sous une humidité relative de 60%, et simultanément, toutes les 12 heures, et pendant une durée de 20 minutes, elles sont soumises à une aspersion d'eau.

### Résistance à la permanente :

Les mèches teintes sont immergées dans une solution réductrice de permanente Dulcia Vital (L'Oréal), de force variant de 1 à 3, pendant une durée variant de 10 à 20 minutes; les mèches sont rincées; on les trempe ensuite dans une solution de fixateur (oxydant), pendant 5 minutes Après rinçage à l'eau, lavage au shampooing standard et rinçage à l'eau, on les sèche.

### EXEMPLE 1 :

On a préparé la composition de teinture, conforme à l'invention, suivante :

| | |
|---|---|
| -2-(β-hydroxyéthyl) paraphénylènediamine, dichlorhydrate | 0,45 g |
| -2-méthyl 5-aminophénol | 0,40 g |
| -Alcool oléique polyglycérolé à 2 moles de glycérol | 4,0 g |
| -Alcool oléique polyglycérolé à 4 moles de glycérol (78 % de MA) | 5,7 g MA |
| -Acide oléique | 3,0 g |
| -Amine oléique à 2 moles d'oxyde d'éthylène, vendue sous la dénomination Ethomeen 012 par la Société AKZO | 7,0 g |
| -Laurylaminosuccinamate de diéthylaminopropyle, sel de sodium à 55% de MA | 3,0 g MA |
| -Alcool oléique | 5,0 g |
| -Diéthanolamide d'acide oléique | 12,0 g |
| -Propylèneglycol | 3,5 g |
| -Alcool éthylique | 7,0 g |
| -Dipropylèneglycol | 0,5 g |
| -Monométhyléther de propylèneglycol | 9,0 g |
| -Métabisulfite de sodium en solution aqueuse à 35% de MA | 0,4 g MA |
| -Acétate d'ammonium | 0,8 g |
| -Antioxydant, séquestrant, | qs |
| -Parfum, conservateur, | qs |
| -Ammoniaque à 20% de NH₃ | 10,0 g |
| -Eau déminéralisée qsp | 100 g |

Au moment de l'emploi, on a mélangé cette composition poids pour poids avec de l'eau oxygénée titrant 20 volumes (6% en poids), de pH 3.

On a obtenu un mélange de pH 9,8.

On a ensuite appliqué ce mélange sur des cheveux gris à 90% de blancs, pendant 30 minutes. Après rinçage, lavage au shampooing, rinçage et séchage, les cheveux sont teints dans une nuance rouge-pourpre qui résiste en particulier remarquablement bien aux shampooings.

## Revendications

1. Composition de teinture d'oxydation pour fibres kératiniques, en particulier pour fibres kératiniques humaines telles que les cheveux, du type comprenant, dans un milieu approprié pour la teinture, au moins un précurseur de colorant d'oxydation et au moins un coupleur, caractérisée par le fait qu'elle contient, à titre de précurseur de colorant d'oxydation, la 2-(β-hydroxyéthyl) paraphénylènediamine et/ou au moins l'un de ses sels d'addition avec un acide, et à titre de coupleur, le 2-méthyl 5-aminophénol et/ou au moins l'un de ses sels d'addition avec un acide,
ladite composition étant exempte de précurseur de colorant d'oxydation additionnel choisi dans le groupe constitué par le 3-méthyl para-aminophénol, le 2-méthyl para-aminophénol et le 2-hydroxyméthyl para-aminophénol.

2. Composition de teinture selon la revendication 1, caractérisée par le fait que les sels d'addition avec un acide sont choisis parmi les chlorhydrates, les sulfates, les bromhydrates et les tartrates.

3. Composition de teinture selon l'une des revendications 1 à 2, caractérisée par le fait que la 2-(β-hydroxyéthyl) paraphénylènediamine ou ses sels est présente dans une concentration comprise entre 0,01 et 10 % en poids environ par rapport au poids total de la composition, de préférence entre 0,05 et 5 % en poids environ, et que le 2-méthyl 5-aminophénol ou ses sels est présent dans une concentration comprise entre 0,005 et 3 % en poids environ, de préférence entre 0,05 et 2 % en poids environ, par rapport au poids total de la composition.

4. Composition de teinture selon l'une quelconque des revendications 1 à 3, prête à l'emploi, caractérisée en ce qu'elle contient en outre un agent oxydant et qu'elle présente un pH compris entre 3 et 11.

5. Procédé de teinture des fibres kératiniques et en particulier des fibres kératiniques humaines telles que les cheveux, caractérisé par le fait qu'il consiste à appliquer sur les fibres une composition de teinture (A) selon l'une quelconque des revendications 1 à 4, et à révéler la couleur en milieu alcalin neutre ou acide à l'aide d'un agent oxydant qui est ajouté juste au moment de l'emploi à cette composition (A) ou qui est présent dans une composition (B) appliquée simultanément ou séquentiellement de façon séparée.

6. Dispositif à plusieurs compartiments ou "Kit" pour la teinture des fibres kératiniques et en particulier des fibres kératiniques humaines telles que les cheveux, caractérisé par le fait qu'il comporte au moins deux compartiments, dont l'un d'entre eux renferme une composition (A) telle que définie dans l'une quelconque des revendications 1 à 3, et un autre une composition (B) comprenant un agent oxydant dans un milieu approprié pour la teinture.

7. Utilisation d'une composition de teinture selon l'une quelconque des revendications 1 à 4 ou d'un dispositif de teinture ou "Kit" à plusieurs compartiments selon la revendication 6, pour la teinture des fibres kératiniques humaines telles que les cheveux.

## Claims

1. Oxidation dye composition for keratinous fibres, in particular for human keratinous fibres such as hair, of the type comprising, in a medium which is suitable for dyeing, at least one oxidation dye precursor and at least one coupling agent, characterized in that it contains, as oxidation dye precursor, 2-(β-hydroxyethyl)-para-phenylenediamine and/or at least one of the addition salts thereof with an acid, and, as coupling agent, 2-methyl-5-aminophenol and/or at least one of the addition salts thereof with an acid, the said composition being free of any additional oxidation dye precursor chosen from the group consisting of 3-methyl-para-aminophenol, 2-methyl-para-aminophenol and 2-hydroxymethyl-para-aminophenol.

2. Dye composition according to Claim 1, characterized in that the addition salts with an acid are chosen from hydrochlorides, sulphates, hydrobromides and tartrates.

3. Dye composition according to either of Claims 1 and 2, characterized in that 2-(β-hydroxyethyl)-para-phenylenediamine, or the salts thereof, is present in a concentration of between 0.01 and 10 % by weight approximately relative to the total weight of the composition, preferably between 0.05 and 5 % by weight approximately, and that 2-methyl-5-aminophenol, or the salts thereof, is present in a concentration of between 0.005 and 3 % by weight approximately, and preferably between 0.05 and 2 % by weight approximately, relative to the total weight of the composition.

4. Dye composition according to any one of Claims 1 to 3, which is a ready-to-use composition, characterized in that it additionally contains an oxidizing agent and in that it has a pH between 3 and 11.

5. Process for dyeing keratinous fibres and in particular human keratinous fibres such as hair, characterized in that it consists in applying to the fibres a dye composition (A) according to any one of Claims 1 to 4, and in developing the colour in an alkaline, neutral or acidic medium using an oxidizing agent which is added only at the time of use to this composition (A) or which is present in a composition (B) that is applied simultaneously or sequentially in a separate manner.

6. Device containing several compartments, or "kit", for dyeing keratinous fibres, and in particular human keratinous fibres such as hair, characterized in that it is composed of at least two compartments, one of which contains a composition (A) as defined in any one of Claims 1 to 3, and another of which contains a composition (B) comprising an oxidizing agent in a medium which is suitable for dyeing.

7. Use of a dye composition according to any one of Claims 1 to 4, or of a dyeing device or "kit" containing several compartments according to Claim 6, for dyeing human keratinous fibres such as hair.

## Patentansprüche

1. Zusammensetzung zum oxidativen Färben von Keratinfasern, insbesondere von menschlichen Keratinfasern, wie dem Haar, die in einem zum Färben geeigneten Medium mindestens ein Farbstoffvorprodukt eines Oxidationsfarbstoffes und mindestens einen Kuppler enthält,
**dadurch gekennzeichnet,** daß sie als Farbstoffvorprodukt eines Oxidationsfarbstoffes 2-(β-Hydroxyethyl)-p-phenylendiamin und/oder mindestens eines seiner Additionssalze mit einer Säure und als Kuppler 2-Methyl-5-aminophenol und/oder mindestens eines seiner Additionssalze mit einer Säure enthält,
wobei die Zusammensetzung kein zusätzliches Farbstoffvorprodukt eines Oxidationsfarbstoffes enthält, das unter 3-Methyl-p-aminophenol, 2-Methyl-p-aminophenol und 2-Hydroxymethyl-p-aminophenol ausgewählt ist.

2. Zusammensetzung zum Färben nach Anspruch 1, dadurch gekennzeichnet, daß die Additionssalze mit einer Säure unter den Hydrochloriden, Sulfaten, Hydrobromiden und Tartraten ausgewählt sind.

3. Zusammensetzung zum Färben nach einem der Ansprüche 1 oder 2, dadurch gekennzeichnet, daß das 2-(β-Hydroxyethyl)-p-phenylendiamin oder seine Salze in einer Konzentration im Bereich von etwa 0,01 bis 10 Gew.-%, bezogen auf das Gesamtgewicht der Zusammensetzung, und vorzugsweise im Bereich von etwa 0,05 bis 5 Gew.-% und das 2-Methyl-5-aminophenol oder seine Salze in einer Konzentration im Bereich von etwa 0,005 bis 3 Gew.-% und vorzugsweise von etwa 0,05 bis 2 Gew.-%, bezogen auf das Gesamtgewicht der Zusammensetzung, vorliegen.

4. Zusammensetzung zum Färben nach einem der Ansprüche 1 bis 3, die anwendungsfertig ist und die dadurch gekennzeichnet ist, daß sie ferner ein Oxidationsmittel enthält und einen pH-Wert von 3 bis 11 aufweist.

5. Verfahren zum Färben von Keratinfasern und insbesondere von menschlichen Keratinfasern, wie dem Haar, dadurch gekennzeichnet, daß es darin besteht, auf die Fasern eine Zusammensetzung zum Färben (A) nach einem der Ansprüche 1 bis 4 aufzutragen und die Farbe im alkalischen, neutralen oder sauren Medium mit einem Oxidationsmittel zu entwickeln, das dieser Zusammensetzung (A) bei der Anwendung zugesetzt wird und das in einer Zusammensetzung (B) vorliegt, die gleichzeitig oder getrennt davon anschließend aufgetragen wird.

6. Vorrichtung mit mehreren Abteilungen oder "Kit" zum Färben von Keratinfasern und insbesondere menschlichen Keratinfasern, wie dem Haar, dadurch gekennzeichnet, daß sie mindestens zwei Abteilungen aufweist, wobei eine Abteilung eine Zusammensetzung (A) nach einem der Ansprüche 1 bis 3 und die andere eine Zusammensetzung (B) enthält, die in einem zum Färben geeigneten Medium ein Oxidationsmittel enthält.

7. Verwendung einer Zusammensetzung zum Färben nach einem der Ansprüche 1 bis 4 oder einer Vorrichtung zum Färben oder eines "Kits" mit mehreren Abteilungen nach Anspruch 6 zum Färben von menschlichen Keratinfasern, wie dem Haar.
